(19) Europäisches Patentamt
European Patent Office
Office européen des brevets

(11) **EP 0 966 950 B1**

(12) **EUROPÄISCHE PATENTSCHRIFT**

(45) Veröffentlichungstag und Bekanntmachung des Hinweises auf die Patenterteilung:
**15.09.2004   Patentblatt 2004/38**

(51) Int Cl.⁷: **A61K 7/11**, A61K 7/50

(21) Anmeldenummer: **99110739.2**

(22) Anmeldetag: **04.06.1999**

(54) **Einphasiges, schaumförmiges Haarbehandlungsmittel zur Erzielung eines Pomadeneffektes**

Monophase effervescent hair treatment composition achieving the effect of a pomade

Composition effervéscente monophasée pour le traitement des cheveux pour réaliser un effet de pommade

(84) Benannte Vertragsstaaten:
**DE ES FR GB IT**

(30) Priorität: **26.06.1998   DE 19828643**

(43) Veröffentlichungstag der Anmeldung:
**29.12.1999   Patentblatt 1999/52**

(73) Patentinhaber: **Wella Aktiengesellschaft**
**64274 Darmstadt (DE)**

(72) Erfinder:
• **Irrgang, Bernhard Dr.**
  **1713 St. Anton (CH)**
• **Karlen, Thomas Dr.**
  **3013 Bern (CH)**

(56) Entgegenhaltungen:
EP-A- 0 559 375          EP-A- 0 681 832
EP-A- 1 043 010

## Beschreibung

Gegenstand der Erfindung ist ein einphasiges, schaumförmiges Haarbehandlungsmittel zur Erzielung eines Pomadeneffektes, insbesondere zur Erzielung von Festigung und Glanz der behandelten Haare.

Schon seit langem beschäftigt sich die Forschung auf dem Gebiet der Haarbehandlungsmittel mit Zubereitungen in Form von Pomaden, welche zur erleichterten Formgebung und zur Erhöhung des Glanzes und des Haltes der Frisur dienen. Die Darreichungsform von Pomaden beschränkt sich üblicherweise weitgehend auf nichttransparente Massen in Form von cremeartigen Emulsionen oder Gelen. Diese Darreichungsformen führen oft zu einer schlechten Verteilbarkeit der Masse auf dem Haar, wodurch die Frisurenerstellung erschwert wird, insbesondere wenn nur kleine Haarpartien mit dem Mittel behandelt werden sollen.

Die zur Erzielung eines Pomadeneffektes, das heißt zur gleichzeitigen Erzielung von intensivem Glanz und Halt verwendeten herkömmlichen Produkte weisen üblicherweise einen hohen Anteil an wasserunlöslichen Stoffen wie z.B. Fettkörpern auf. Durch diese Fettkörper wird aber das Haar stark belastet und das Mittel ist nur schwer durch Shampoonieren rückstandslos wieder zu entfernen. Außerdem besteht bei derartigen Produkten das Problem der unerwünschten Separierung von Wasser- und Fettstoffphase.

Eine verbesserte Applikationsmöglichkeit bietet sich dadurch an, daß die Pomade in Form eines Schaums auf das Haar aufgetragen wird, der beim Einarbeiten in das Haar beziehungsweise beim Erstellen der Frisur zusammenbricht.

Die Aufgabe der vorliegenden Erfindung bestand darin, ein Mittel zur Verfügung zu stellen, mit welchem ein intensiver Pomadeneffekt erzielt werden kann, wobei die für klassische Pomaden typischen negativen Eigenschaften wie z.B. schlechte Verteilbarkeit auf dem Haar, starke Belastung der Haare, schlechte Auswaschbarkeit der Haare und Phasenauftrennung des Produktes nach Möglichkeit vermieden werden sollen.

Niedrig- bis mittelviskose Massen mit den fachlichen Eigenschaften einer Pomade können mit Hilfe eines Aerosol-Schaums appliziert werden. Der Vorteil eines treibgashaltigen Aerosol-Produktes ist die sehr einfache Erzeugung von Schaum auch mit geringen Mengen an nur schwach schäumenden Inhaltsstoffen. Nachteilig ist jedoch, daß sich das schwierige Problem stellt, zur Konfektionierung als einphasiges Produkt das Treibgas in der wässrigen Phase zu stabilisieren. Eine Phasenseparation kann außer der aesthetischen Einheit des Produktes die fachlichen Eigenschaften verändern. Soll das Aerosolprodukt in einer transparenten Verpackung enthalten sein, so kommt als Verpakkungsmaterial im wesentlichen nur Glas in Betracht. Die Verpackung eines Aerosolproduktes in einer Glasverpackung stellt aber ein potentielles Sicherheitsrisiko dar.

Die Verschäumung ohne Treibgas gelingt einfacherweise durch Verwendung von stark schäumenden Tensiden aus der Klasse der Aniontenside, Amphotenside oder nichtionischer Tenside. Der Nachteil bei der Verwendung dieser Tenside in Konzentrationen, welche für eine zu verschäumende Pomadengrundlage notwendig sind, liegt darin, daß bei der Einarbeitung ins Haar der Schaum nicht zusammenbricht, sondern daß vielmehr ein Nachschäumen, vergleichbar mit einem Shampoo, auf dem Haar stattfindet, was für die Applikation einer Pomade unerwünscht ist. Zudem sind Pomadenmassen, welche größere Anteile an diesen Tensiden enthalten, zu viskos, um problemlos ohne Hilfe von Treibgas eine gute Verschäumbarkeit zu gewährleisten. Eine Verdünnung mit organischen Lösungsmitteln führt zwar zu einer Erniedrigung der Viskosität, bringt aber auch eine drastische Verschlechterung der Verschäumbarkeit mit sich. Wenn mit Wasser verdünnt wird, bis eine leicht zu verschäumende Viskosität der Masse erreicht ist, führt dies zu einer Verschlechterung des Pomadeneffektes.

Gelöst wird die Aufgabe durch ein Mittel zur Haarbehandlung bestehend aus

    (A) einer einphasigen Zusammensetzung enthaltend

        (a1) mindestens ein anionisches, eine Carboxylatgruppe enthaltendes Tensid und
        (a2) mindestens einen nichtionischen Emulgator in Kombination mit

    (B) einer Vorrichtung zum Verschäumen der Zusammensetzung (A).

Das Mittel läßt sich auch ohne Treibgas ausgezeichnet verschäumen und läßt sich ohne störendes Nachschäumen gut in das Haar einarbeiten. Es verleiht der Frisur eine gute Formbarkeit, einen langanhaltenden Glanz und einen langanhaltenden weichen Griff.

Das eine Carboxylatgruppe enthaltende Tensid ist vorzugsweise in einer Menge von 0,1 bis 30 Gewichtsprozent, besonders bevorzugt in einer Menge von 1 bis 10 Gewichtsprozent enthalten und ist insbesondere ausgewählt aus Sulfosuccinaten, Sulfosuccinamaten, carboxylierten Fettalkoholethoxylaten oder carboxylierten Fettsäureamidethoxylaten oder deren Gemischen.

Vorzugsweise besitzt die Komponente (a1) die allgemeine Formel (I)

$$R^1\text{-X-(CHR}^2\text{-CHR}^3\text{-O)}_n\text{-Y-CO}_2^- \ 1/m \ M^{m+} \tag{I}$$

wobei -X- für -COO-, -CONH-, -O- oder -NH- steht; -Y- für eine Alkylengruppe mit vorzugsweise 1 bis 4 Kohlenstoffatomen, insbesondere für eine Methylengruppe steht oder -Y-CO$_2^-$ für eine Sulfosuccinatgruppe steht; R$^1$ für eine gesättigte oder ungesättigte, verzweigte oder unverzweigte Kohlenwasserstoffgruppe mit vorzugsweise 5 bis 20 Kohlenstoffatomen, die mit Hydroxygruppen substituiert sein kann oder R$^1$-X für eine alkoxylierte oder nichtalkoxylierte Alkylcitratgruppe steht; R$^2$ und R$^3$ unabhängig voneinander für Wasserstoff oder für eine Methylgruppe stehen; n den Alkoxylierungsgrad bedeutet, der zwischen 0 und 20, vorzugsweise zwischen 0 und 10 liegt; M für ein oder mehrere, die negativen Ladungen des Anions neutralisierende Gegenionen, beipielsweise ein Metallion oder ein quaternäres Ammoniumion steht und m die Wertigkeit des Gegenions M bedeutet. Steht -Y-CO$_2^-$ für eine Sulfosuccinatgruppe, so ist diese vorzugsweise von der Form -C(O)-CHR$^4$-CHR$^5$-CO$_2^-$, wobei einer der Reste R$^4$ und R$^5$ Wasserstoff und der andere SO$_3^-$ bedeutet. Steht R'-X für eine alkoxylierte oder nichtalkoxylierte Alkylcitratgruppe, so ist diese vorzugsweise von der Form

$$R^6\text{O-(CH2CH2O)}_{n1}\text{-CO-CH2-C(OH)(CO- (OCH2CH2)}_{n2}\text{OH)-CH2-COO-,}$$

wobei R$^6$ für eine gesättigte oder ungesättigte, verzweigte oder unverzweigte Kohlenwasserstoffgruppe mit vorzugsweise 5 bis 20 Kohlenstoffatomen, die mit Hydroxygruppen substituiert sein kann, steht und n1 und n2 den Alkoxylierungsgrad bedeuten, der jeweils zwischen 0 und 10, vorzugsweise zwischen 0 und 5 liegt.

Als Komponente (a1) geeignete, eine Carboxylatgruppe enthaltende Tenside sind insbesondere

- Verbindungen der allgemeinen Formel (II)

$$R^1\text{-X-(CHR}^2\text{-CHR}^3\text{-O)}_n\text{-C(O)-CHR}^4\text{-CHR}^5\text{-CO}_2^- \ 1/m \ M^{m+} \tag{II}$$

wobei R$^2$, R$^3$, n, m und M die oben angegebenen Bedeutungen haben und wobei mindestens einer der Reste R$^2$ und R$^3$ Wasserstoff bedeutet; R$^1$ für eine gesättigte oder ungesättigte, verzweigte oder unverzweigte Kohlenwasserstoffgruppe mit vorzugsweise 5 bis 20 Kohlenstoffatomen, die mit Hydroxygruppen substituiert sein kann, steht; -X- für -COO-, -CONH- oder -NHsteht und die Reste R$^4$ und R$^5$ unabhängig voneinander Wasserstoff oder SO$_3^-$ bedeuten, wobei mindestens einer der beiden Reste Wasserstoff bedeutet.

Geeignete Sulfosuccinate sind beispielsweise bekannt unter den INCI-Bezeichnungen Disodium Ricinoleamido MEA-Sulfosuccinate, Disodium Cocamido MIPA-Sulfosuccinate, Disodium PEG-4 Cocamido MIPA-Sulfosuccinate, Disodium PEG-5 Laurylcitratesulfosuccinate, Disodium Cocamido PEG-3 Sulfosuccionate, Disodium Laneth-5 Sulfosuccinate oder Disodium Undecyleneamido MEA-Sulfosuccinate. Ein geeignetes Sulfosuccinamat ist beispielsweise bekannt unter der INCI-Bezeichnung Disodium Tallow Sulfosuccinamate.

- Alkylcitrate der allgemeinen Formel (III)

$$R^6\text{O-(CH2CH2O)}_{n1}\text{-CO-CH2-C(OH)(CO-(OCH2CH2)}_{n2}\text{OH)-CH2-}$$

$$\text{-CO-(OCH2CH2)}_{n3}\text{-O-CO-CHR}^4\text{-CHR}^5\text{-CO}_2^- \ 1/m \ M^{m+} \tag{III}$$

wobei m und M die oben angegebenen Bedeutungen haben; R$^6$ für eine gesättigte oder ungesättigte, verzweigte oder unverzweigte Kohlenwasserstoffgruppe mit vorzugsweise 5 bis 20 Kohlenstoffatomen, die mit Hydroxygruppen substituiert sein kann, steht; die Reste R$^4$ und R$^5$ unabhängig voneinander Wasserstoff oder SO$_3^-$ bedeuten, wobei mindestens einer der beiden Reste Wasserstoff bedeutet und n1, n2 und n3 den Alkoxylierungsgrad bedeuten, der jeweils zwischen 0 und 10, vorzugsweise zwischen 0 und 5 liegt.

Ein geeignetes Alkylcitrat-Sulfosuccinat ist beispielsweise bekannt unter der INCI-Bezeichnung Disodium PEG-10 Laurylcitratesulfosuccinate oder Disodium PEG-5 Laurylcitrate Sulfosuccinate (REWOPOL® SB CS 50 der Firma Witco Surfactants GmbH, Deutschland).

- Verbindungen der allgemeinen Formel (IV)

$$R^1\text{-X-(CH2CH2O)}_n\text{-CH2-CO}_2^-\ 1/m\ M^{m+} \tag{IV}$$

wobei n, m und M die oben angegebenen Bedeutungen haben; $R^1$ für eine gesättigte oder ungesättigte, verzweigte oder unverzweigte Kohlenwasserstoffgruppe mit vorzugsweise 5 bis 20 Kohlenstoffatomen, die mit Hydroxygruppen substituiert sein kann, steht und -Xfür -COO-, -CONH- oder -O- steht, insbesondere carboxylierte Fettalkoholethoxylate und Fettsäureamidethoxylate.

Ein geeignetes carboxyliertes Fettalkoholethoxylat ist beispielsweise bekannt unter der INCI-Bezeichnung Sodium Laureth-13 Carboxylat.

Beispiele geeigneter carboxylierter Fettalkoholethoxylate oder Fettsäureamidethoxylate sind Akypo Soft®-Typen der Firma Chem-Y, Miranate® LEC oder Sandopan® LS-24 (INCI: Natrium Laureth-13 Carboxylat) der Firma Rhone Poulenc oder der Firma Clariant.

Ein weiterer Gegenstand der Erfindung ist die Verwendung eines anionischen, eine Carboxylatgruppe enthaltenden Tensids zur Herstellung einer transparenten, einphasigen, mittels einer mechanischen Vorrichtung verschäumbaren Zusammensetzung, wobei das eine Carboxylatgruppe enthaltende Tensid vorzugsweise ein Sulfosuccinat, ein Sulfosuccinamat, ein carboxyliertes Fettsäureamidethoxylat oder ein carboxyliertes Fettalkoholethoxylat ist, insbesondere ein eine Carboxylatgruppe enthaltendes Tensid der allgemeinen Formel (I).

Geeignete nichtionische Emulgatoren sind beispielsweise die im "International Cosmetic Ingredient Dictionary and Handbook", 7. Auflage, Band 2 im Abschnitt "Surfactants - Emulsifying Agents" aufgeführten nichtionischen Emulgatoren. Die als Komponente a2 geeigneten nichtionischen Emulgatoren sind vorzugsweise ausgewählt aus ethoxylierten Fettsäuren mit 10 bis 26 Kohlenstoffatomen, ethoxylierten ein- oder mehrwertigen Alkoholen mit 1 bis 6 Kohlenstoffatomen, ethoxylierten Fettalkoholen mit 10 bis 26 Kohlenstoffatomen, ethoxyliertem hydriertem oder nicht hydriertem Rizinusöl, Glyceridalkoxylaten, Fettsäureglyceridpolyalkylenglykolethern oder Fettsäurepartialglyceridpolyalkylenglykolethern mit jeweils weniger als 30 Alkylenglykoleinheiten wie beispielsweise Polyethylenglykol(7)-glycerylcocoat, Polyglykolamiden, Fettsäurezuckerestern, ethoxylierten Fettsäurezuckerestern und Partialglyceriden. Der Ethoxylierungsgrad von ethoxylierten Tensiden beträgt üblicherweise von 1 bis 400 und ist vorzugsweise größer als 3.

Geeignete ethoxylierte Fettsäuren sind beispielsweise Polyethylenglykol(75)-laurat, Polyethylenglykol(90)-stearat, Polyethylenglykol(120)-stearat, Polyethylenglykol(120)-propylenglykolstearat, Polyethylenglykol(150)-dilaurat oder Polyethylenglykol(175)-distearat.

Geeignete ethoxylierte Fettsäurezuckerester sind zum Beispiel ethoxylierte Sorbitanfettsäureester oder Polyethylenglykol(120)-methylglucosedioleat.

Bei den Partialglyceriden kann es sich um Mono- oder Diglyceride oder um Mischungen aus Mono- und Diglyceriden handeln. Beispiele für geeignete Verbindungen sind Polyethylenglykol(30)-glycerylcocoat, Polyethylenglykol(80)-glycerylcocoat, Polyethylenglykol(80)-glyceryltallowat, Polyethylenglykol(120)-glycerylstearat, Polyethylenglykol(200)-glycerylstearat, Polyethylenglykol(200)-glyceryltallowat, hydriertes Polyethylenglykol(200)-glycerylpalmitat. Besonders bevorzugt ist hydriertes Polyethylenglykol(200)-glycerylpalmitat.

In einer bevorzugten Ausführungsform sind in dem erfindungsgemäßen Mittel nur solche Tenside und Emulgatoren enthalten, welche wasserlöslich sind, d.h. solche Tenside, die bei einem Gehalt von 1 Gewichtsprozent in Wasser bei 20°C klar löslich sind. Bevorzugt ist ferner ein Mittel, welches in transparenter, klarer, einphasiger Form und in einer transparenten Verpackung vorliegt. Als Verpackungsmaterial kommt neben Glas vorzugsweise bruchsicherer, transparenter Kunststoff in Betracht.

Das Mittel wird ohne Hilfe von Treibgasen oder chemischen Treibmitteln verschäumt und als Schaum ins Haar eingearbeitet und ohne Ausspülen im Haar belassen.

Das erfindungsgemäße Mitttel weist als Komponente (B) eine insbesondere mechanische Vorrichtung zum Verschäumen der Zusammensetzung (A) auf. Unter mechanischen Schäumvorrichtungen sind solche Vorrichtungen zu verstehen, welche das Verschäumen einer Flüssigkeit ohne Verwendung eines Treibmittels ermöglichen. Als geeignete mechanische Schäumvorrichtung kann beispielsweise ein handelsüblicher Pumpschäumer verwendet werden.

Geeignete Vorrichtungen zum Verschäumen sind zum Beispiel beschrieben in der EP 0 736 462 und der darin zitierten Literatur und sind erhältlich beispielsweise von der Firma Yoshino Kogyosho Co., Ltd., Japan. Besonders bevorzugt ist der Pumpschäumer Daiwa F2 der Firma Yoshino Kogyosho.

In der erfindungsgemäßen Zusammensetzung (A) können desweiteren Lösungsmittel mit einem Siedepunkt unter 600 °C eingesetzt werden. In einer bevorzugten Ausführungsform enthält das erfindungsgemäße Mittel mindestens einen mehrwertigen Alkohol, insbesondere einen mit 2 bis 4 Kohlenstoffatomen wie beispielsweise Propylenglykol oder Glycerin. Die Lösungsmittel liegen in einer Menge von 0,01 bis 50 Gewichtsprozent, bevorzugt in einer Menge von 2 bis 30 Gewichtsprozent vor.

Das erfindungsgemäße Mittel kann zusätzlich Alkohole, insbesondere die für kosmetische Zwecke üblicherweise verwendeten niederen Alkohole mit 1 bis 4 Kohlenstoffatomen wie zum Beispiel Ethanol oder Isopropanol enthalten in kleinen Mengen bis zu 10 Gewichtsprozent enthalten. Bevorzugt sind jedoch Mittel, welche keine Alkohole enthalten.

Ebenfalls enthalten sein können bei Raumtemperatur flüssige, wachsartige oder feste Polyethylenglykole oder Copolymere zwischen Ethylenglykol und Propylenglykol.

Das erfindungsgemäße Mittel kann zusätzlich wasserunlösliche Lösungsmittel enthalten, beispielsweise unverzweigte oder verzweigte Kohlenwasserstoffe wie Pentan, Hexan, Isopentan und cyclische Kohlenwasserstoffe wie Cyclopentan, Cyclohexan, Paraffine sowie Isododecan. Bevorzugte Anwendungen des erfindungsgemäßen Mittels enthalten jedoch keine solchen wasserunlöslichen Zusätze.

Als weiteren zusätzlichen Bestandteil kann das erfindungsgemäße Haarbehandlungsmittel mindestens ein filmbildendes, haarfestigendes Polymer in einer Menge von vorzugsweise 0,01 bis 10 Gewichtsprozent, besonders bevorzugt von 0,1 bis 8 Gewichtsprozent enthalten. Das Polymer kann synthetischen oder natürlichen Ursprungs und nichtionischer, anionischer oder amphoterer Natur sein. Die haarfestigenden Polymere können einzeln oder im Gemisch eingesetzt werden.

Unter filmbildenden, haarfestigenden Polymeren werden erfindungsgemäß solche Polymere verstanden, die bei Anwendung in 0,1 bis 5%iger wäßriger, alkoholischer oder wäßrig-alkoholischer Lösung in der Lage sind, auf dem Haar einen Polymerfilm abzuscheiden und auf diese Weise das Haar zu festigen.

Enthalten die Polymere Säuregruppen, so können diese teilweise oder ganz mit einer geeigneten organischen oder anorganischen Base neutralisiert werden. Bevorzugte Basen sind primäre und sekundäre Amine, insbesondere Alkanolamine wie beispielsweise Aminomethylpropanol. Enthalten die Polymere basische Gruppen, so können diese teilweise oder ganz mit einer geeigneten Säure wie z.B. Ameisensäure, Pyrrolidoncarbonsäure, Milchsäure usw. neutralisiert werden.

Als geeignete synthetische, nichtionische, filmbildende, haarfestigende Polymere können in dem erfindungsgemäßen Haarbehandlungsmittel Homopolymere des Vinylpyrrolidons, Homopolymere des N-Vinylformamids, Copolymere des Vinylpyrrolidons und Dimethylaminoalkylmethacrylaten, wobei Alkyl Methyl Ethyl oder Propyl bedeuten kann, Copolymerisate aus Vinylpyrrolidon und Vinylacetat, Terpolymere aus Vinylpyrrolidon, Vinylacetat und Vinylpropionat, Polyacrylamide, Polyvinylalkohole, oder Polyethylenglykol/Polypropylenglykol Copolymere eingesetzt werden.

Geeignete synthetische filmbildende, anionische Polymere sind z.B. vernetzte oder unvernetzte Vinylacetat/Crotonsäure Copolymere, Vinylpyrrolidon/Vinylacrylat Copolymere, Terpolymere aus Acrylsäure, Alkylacrylat und N-Alkylacrylamid, insbesondere Acrylsäure/Ethylacrylat/ N-t-Butylacrylamid Terpolymere oder Terpolymere aus Vinylacetat, Crotonat und Vinylalkanoat, insbesondere Vinylacetat/Crotonat/Vinylneodecanoat Copolymere sowie Methylvinylether/Maleinsäureanhydrid Copolymere und deren Monoester.

Natürliche filmbildende Polymere oder daraus durch chemische Umwandlung hergestellte Derivate können in dem erfindungsgemäßen Haarbehandlungsmittel ebenfalls eingesetzt werden. Bewährt haben sich Polysaccharide oder Gemische aus Oligo-, Mono- und Disacchariden (C-Pur® 01924 von Cerestar), chinesisches Balsamharz (Kolophonium), Cellulosederivate wie Hydroxypropylcellulose mit einem Molekulargewicht von 30.000 bis 50.000 g/mol, oder Schellack in neutralisierter oder unneutralisierter Form.

Auch amphotere Polymere können in dem erfindungsgemäßen Haarbehandlungsmittel eingesetzt werden. Amphotere Polymere besitzen im Molekül entweder sowohl freie basische Gruppen wie zum Beispiel Aminogruppen und freie saure Gruppen wie zum Beispiel Carbonsäure- oder Sulfonsäuregruppen und sind zur Bildung von inneren Salzen befähigt oder sie enthalten sowohl kationische Gruppen wie zum Beispiele quaternäre Ammoniumgruppen und anionische Gruppen wie beispielsweise Carboxylat- oder Sulfat- oder Sulfonatgruppen. Geeignet sind insbesondere Copolymere gebildet aus Alkylacrylamid, insbesondere Octylacrylamid, Alkylaminoalkylmethacrylat, insbesondere t-Butylaminoethylmethacrylate und zwei oder mehr Monomeren bestehend aus Acrylsäure, Methacrylsäure oder deren Ester, wie sie zum Beispiel unter dem Handelsnamen Resyn 28-4910 oder Amphomer LV-71 der Firma NATIONAL STARCH, USA erhältlich sind.

Desweiteren kann das erfindungsgemäße Mittel wasserlösliche oder wasserunlösliche Silikonverbindungen in einer Konzentration von 0,01 bis 50 Gewichtsprozent, bevorzugt in einer Konzentration von 0,1 bis 5 Gewichtsprozent enthalten. Besonders bevorzugt sind dabei flüchtige und nichtflüchtige Cyclomethicone und Dimethicone sowie Dimethicon-Copolyole. Beispiele sind: Polydimethylsiloxan (Dimethicon), $\alpha$-Hydro-$\omega$-hydroxypolyoxydimethylsilylen (Dimethiconol), cyclisches Dimethylpolysiloxan (Cyclomethicon), Trimethyl-(octadecyloxy)silan (Stearoxytrimethylsilan), Dimethylsiloxan/Glykol Copolymer (Dimethicon Copolyol), Dimethylsiloxan/Aminoalkylsiloxan Copolymer mit Hydroxyendgruppen (Amodimethicon), Monomethylpolysiloxan mit Laurylseitenketten und Polyoxyethylen- und/oder Polyoxypropylenendketten, (Laurylmethicon Copolyol), Dimethylsiloxan/Glykol Copolymeracetat (Dimethiconcopolyol Acetat) Dimethylsiloxan/Aminoalkylsiloxan Copolymer mit Trimethylsilylendgruppen (Trimethylsilylamodimethicon). Bevorzugte Silikonpolymere sind: Dimethicone, welche beispielsweise von der Firma Wacker, München, unter der Handelsbezeichnung Siloxane F-221 oder von der Firma Dow Corning Europe, Brüssel, unter der Handelsbezeichnung Dow Corning Fluid 200/0,65 cs vertrieben werden; Cyclomethicone, die beispielsweise unter den Handelsbezeichnungen Dow Corning 244 Fluid von der Firma Dow Corning Europe oder Abil® K4 von der Firma Goldschmidt vertrieben werden; Dimethiconole, die beispielsweise unter den Handelsbezeichnungen Silicone Fluid F-212 von der Firma Wakker oder Unisil® SF-R von der Firma UPI vertrieben werden.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet, wie z.B. eine Mischung aus Dimethicon und Dimethiconol, die beispielsweise unter der Handelsbezeichnung Dow Corning 1403 Fluid von der Firma Dow Corning Europe vertrieben wird.

Selbstverständlich kann das erfindungsgemäße Mittel auch weitere übliche kosmetische Zusätze, wie nichtfestigende, nichtionische Polymere, nichtfestigende, anionische Polymere und nichtfestigende, natürliche Polymere sowie deren Kombination in einer Menge von vorzugsweise 0,01 bis 15 Gewichtsprozent; Parfümöle in einer Menge von vorzugsweise 0,01 bis 5 Gewichtsprozent; Trübungsmittel wie z.B. Ethylenglykoldistearat, Styrol/PVP Copolymere oder Polystyrole in einer Menge von vorzugsweise 0,01 bis 5 Gewichtsprozent; Netzmittel, Tenside oder Emulgatoren mit oder ohne Waschaktivität aus den Klassen der anionischen, kationischen, amphoteren oberflächenaktiven Substanzen wie Fettalkoholsulfate, Fettalkoholethersulfate, Fettsäurealkanolamide in einer Menge von vorzugsweise 0,1 bis 20 Gewichtsprozent; ferner Feuchthaltemittel, Farbstoffe, Lichtschutzmittel, Antioxidantien, Glanzgeber und Konservierungsstoffe in einer Menge von vorzugsweise 0,01 bis 10 Gewichtsprozent enthalten.

Die nachfolgenden Beispiele sollen den Gegenstand der Erfindung näher erläutern.

**Beispiele**

**Beispiel 1: Haarbehandlungsmittel zur erleichterten Formgebung**

| | |
|---|---|
| 8,00 g | Dinatrium PEG-5 Laurylcitrat Sulfosuccinat (REWOPOL® SB CS 50) |
| 15,00 g | Hydriertes Rizinusöl, ethoxyliert mit 40 mol Ethylenoxid (Cremophor® RH 410) |
| 77,00 g | Wasser |
| 100,00 g | |

Das Mittel läßt sich ohne Hilfe eines Treibmittels mit einem Pumpschäumer zu einem feinporigen metastabilen Schaum verschäumen, der beim Einarbeiten ins Haar restlos zusammenbricht und dem Haar Glanz und Halt verleiht.

**Beispiel 2: Haarbehandlungsmittel zur erleichterten Formgebung**

| | |
|---|---|
| 8,00 g | Dinatrium PEG-5 Laurylcitrat Sulfosuccinat (REWOPOL® SB CS 50) |
| 5,00 g | Glycerin |
| 15,00 g | Hydriertes Rizinusöl, ethoxyliert mit 40 mol Ethylenoxid (Cremophor® RH 410) |
| 72,00 g | Wasser |
| 100,00 g | |

**Beispiel 3: Haarbehandlungsmittel zur erleichterten Formgebung**

| | |
|---|---|
| 8,00 g | Dinatrium Ricinoleamido MEA-Sulfosuccinat (REWODERM® S 1333) |
| 5,00 g | Propylenglykol |
| 15,00 g | Hydriertes Rizinusöl, ethoxyliert mit 40 mol Ethylenoxid (Cremophor® RH 410) |
| 72,00 g | Wasser |
| 100,00 g | |

**Beispiel 4: Haarbehandlungsmittel zur erleichterten Formgebung**

| | |
|---|---|
| 8,00 g | Dinatrium PEG-5 Laurylcitrat Sulfosuccinat (REWOPOL® SB CS 50) |
| 5,00 g | Propylenglykol |
| 15,00 g | Hydriertes Rizinusöl, ethoxyliert mit 25 mol Ethylenoxid (Arlatone® G) |
| 72,00 g | Wasser |
| 100,00 g | |

**Beispiel 5: Haarbehandlungsmittel zur erleichterten Formgebung**

| | |
|---|---|
| 8,00 g | Dinatrium PEG-5 Laurylcitrat Sulfosuccinat (REWOPOL® SB CS 50) |
| 5,00 g | Propylenglykol |
| 12,00 g | Hydriertes Rizinusöl, ethoxyliert mit 25 mol Ethylenoxid (Arlatone® G) |
| 1,00 g | Hydriertes Glyceryl Palmitat, ethoxyliert mit 200 mol Ethylenoxid (REWODERM® LI S 80) |
| 74,00 g | Wasser |
| 100,00 g | |

**Beispiel 6: Haarbehandlungsmittel zur erleichterten Formgebung**

| | |
|---|---|
| 8,00 g | Dinatrium PEG-5 Laurylcitrat Sulfosuccinat (REWOPOL® SB CS 50) |
| 5,00 g | Propylenglykol |
| 15,00 g | Cremophor® RH 455 |
| 72,00 g | Wasser |
| 100,00 g | |

**Beispiel 7: Haarbehandlungsmittel zur erleichterten Formgebung**

| | |
|---|---|
| 8,00 g | Disodium PEG-4 Cocamido MIPA-Sulfosuccinate (REWOPOL® SBZ) |
| 5,00 g | Propylenglykol |
| 15,00 g | Hydriertes Rizinusöl, ethoxyliert mit 40 mol Ethylenoxid (Cremophor® RH 410) |
| 72,00 g | Wasser |
| 100,00 g | |

**Beispiel 8: Haarbehandlungsmittel zur erleichterten Formgebung**

| | |
|---|---|
| 8,00 g | Disodium Cocamido PEG-3 Sulfosuccinate (Beaulight® A 5000 S) |
| 5,00 g | Propylenglykol |
| 15,00 g | Hydriertes Rizinusöl, ethoxyliert mit 40 mol Ethylenoxid (Cremophor® RH 410) |
| 1,00 g | Cremophor® EL |
| 71,00 g | Wasser |
| 100,00 g | |

**Beispiel 9: Haarbehandlungsmittel zur erleichterten Formgebung**

| | |
|---|---|
| 8,00 g | Disodium Laureth-3 Sulfosuccinate (Texapon® SB 3) |
| 5,00 g | Propylenglykol |
| 15,00 g | Hydriertes Rizinusöl, ethoxyliert mit 40 mol Ethylenoxid (Cremophor® RH 410) |
| 72,00 g | Wasser |
| 100,00 g | |

**Beispiel 10:Haarbehandlungsmittel zur erleichterten Formgebung**

| | |
|---|---|
| 8,00 g | Magnesium Laureth-11 Carboxylat (Akypo Soft® 100 MgV) |
| 5,00 g | Propylenglykol |
| 15,00 g | Hydriertes Rizinusöl, ethoxyliert mit 40 mol Ethylenoxid (Cremophor® RH 410) |
| 72,00 g | Wasser |
| 100,00 g | |

**Beispiel 11:Haarbehandlungsmittel zur erleichterten Formgebung**

| | |
|---|---|
| 8,00 g | Natrium Laureth-6 Carboxylat (Akypo Soft® 45 NV) |
| 5,00 g | Propylenglykol |
| 15,00 g | Hydriertes Rizinusöl, ethoxyliert mit 40 mol Ethylenoxid (Cremophor® RH 410) |
| 72,00 g | Wasser |
| 100,00 g | |

**Beispiel 12:Haarbehandlungsmittel zur erleichterten Formgebung**

| | |
|---|---|
| 8,00 g | Natrium Laureth-11 Carboxylat (Akypo Soft® 100 BVC) |
| 5,00 g | Propylenglykol |
| 15,00 g | Hydriertes Rizinusöl, ethoxyliert mit 40 mol Ethylenoxid (Cremophor® RH 410) |
| 1,00 g | Tween® 40 |
| 71,00 g | Wasser |
| 100,00 g | |

**Beispiel 13:Haarbehandlungsmittel zur erleichterten Formgebung**

| | |
|---|---|
| 8,00 g | Natrium Laureth-17 Carboxylat (Akypo Soft® 160 NV) |
| 5,00 g | Propylenglykol |
| 15,00 g | Hydriertes Rizinusöl, ethoxyliert mit 40 mol Ethylenoxid (Cremophor® RH 410) |
| 72,00 g | Wasser |
| 100,00 g | |

**Beispiel 14:Haarbehandlungsmittel zur erleichterten Formgebung**

| | |
|---|---|
| 8,00 g | Natrium Laureth-13 Carboxylat (Miranate® LEC oder Sandopan® LS-24) |
| 5,00 g | Propylenglykol |
| 15,00 g | Hydriertes Rizinusöl, ethoxyliert mit 40 mol Ethylenoxid (Cremophor® RH 410) |
| 1,00 g | Cremophor® EL |
| 71,00 g | Wasser |
| 100,00 g | |

**Beispiel 15:Haarbehandlungsmittel zur erleichterten Formgebung**

| | |
|---|---|
| 8,00 g | Disodium PEG-4 Cocamido MIPA-Sulfosuccinate (REWOPOL® SBZ) |
| 5,00 g | Propylenglykol |
| 15,00 g | Hydriertes Rizinusöl, ethoxyliert mit 40 mol Ethylenoxid (Cremophor® RH 410) |
| 1,00 g | Surfactant 193 (Dimethicone Copolyol) |
| 71,00 g | Wasser |
| 100,00 g | |

**Beispiel 16: Vergleich verschiedener Tensidarten**

Die folgenden Zusammensetzungen wurden auf ihre fachlichen Eigenschaften hin untersucht. Beurteilt wurden die Verschäumbarkeit mit Hilfe eines Pumpschäumers, Schaumqualität 15 Sekunden nach dem Verschäumen, unerwünschtes Nachschäumen beim Einarbeiten ins Haar, Haltbarkeit der Frisur und Glanz. Die Benotung erfolgte durch die Bewertung der Einzelkriterien von 5 unabhängigen Personen, wobei die Note 1 sehr gut und die Note 6 unbrauchbar bedeutet.
Alle Angaben beziehen sich auf Aktivgehalt in der Rezeptur.

Verwendete Rohstoffe:

(1) Arlatone® G
(2) Rewoderm® S 1333
(3) Ampho Betain AM®
(4) Texapon® N 70
(5) Oramix® NS 10

Tabelle 1:

| Aktivgehalt Tensid plus Emulgator konstant (außer F). | | | | | | |
|---|---|---|---|---|---|---|
| | A | B | C | D | E | F |
| PEG-25 Hydrogenated Castor Oil (1) | 25 | 18 | 18 | 18 | 18 | 18 |
| Disodium Ricinoleamido MEA-Sulfosuccinate (2) | 0 | 7 | 0 | 0 | 0 | 0 |
| Cocamodopropylbetain (3) | 0 | 0 | 7 | 0 | 0 | 0 |
| Laurylethersulfat 28% (4) | 0 | 0 | 0 | 7 | 0 | 0 |
| Decyl Glucoside (5) | 0 | 0 | 0 | 0 | 7 | 0 |
| Glycerin 86% | 5 | 5 | 5 | 5 | 5 | 5 |
| Wasser | 70 | 70 | 70 | 70 | 70 | 77 |

Tabelle 2:

| Bewertung der fachlichen Eigenschaften | A | B | C | D | E | F |
|---|---|---|---|---|---|---|
| Verschäumbarkeit | 3 | 1 | 2 | 3 | 3 | 2 |
| Schaumqualität nach 15 Sekunden | 4 | 2 | 2 | 2 | 3 | 2 |
| Nachschäumen beim Einarbeiten | 1 | 1 | 6 | 6 | 6 | 1 |
| Halt der Frisur | 1 | 1 | 4 | 3 | 3 | 4 |
| Glanz | 2 | 1 | 6 | 6 | 6 | 4 |

Bei dem Kriterium "Schaumqualität nach 15 Sekunden" erhält ein Schaum eine gute Bewertung, wenn er 15 Sekunden nach der Entnahme und vor Einarbeitung in das Haar möglichst feinporig und möglichst wenig zusammengefallen ist. Muster C, D und E schäumten beim Einarbeiten ins Haar sehr stark nach (Shampoo Effekt), sodaß ein Reinigen der Haare nötig wurde.

Die dem erfindungsgemäßen Mittel entsprechende Rezeptur B verbessert die fachlichen Eigenschaften der nur aus einem nichtionischen Tensid bestehenden Rezeptur A in wesentlichen Punkten und besitzt keine Nachteile der Rezepturen C, D und E.

Werden die Rezepturen B,C,D und E mit einer Rezeptur F verglichen, bei der der Aktivgehalt an Arlatone® G demjenigen der Rezepturen B bis E entspricht, so kann man feststellen, daß Halt und Glanz der Frisur, welche mit dieser Rezeptur behandelt wurden, schlechter sind als diejenigen, welche mit Rezeptur B behandelt wurden.

**Patentansprüche**

1.  Mittel zur Haarbehandlung bestehend aus

    (A) einer einphasigen Zusammensetzung enthaltend

        (a1) mindestens ein anionisches, eine Carboxylatgruppe enthaltendes Tensid und
        (a2) mindestens einen nichtionischen Emulgator in Kombination mit

    (B) einer Pumpschäumvorrichtung zum Verschäumen der Zusammensetzung (A).

2.  Mittel nach Anspruch 1, **dadurch gekennzeichnet, daß** Komponente (a1) ein Sulfosuccinat-Tensid ist.

3.  Mittel nach Anspruch 1, **dadurch gekennzeichnet, daß** Komponente (a1) die allgemeine Formel (I) aufweist

$$R^1\text{-X-}(CHR^2\text{-}CHR^3\text{-O})_n\text{-Y-}CO_2^{-}\ 1/m\ M^{m+} \tag{I}$$

wobei -X- für -COO-, -CONH-, -O- oder -NH- steht; -Y- für eine Alkylengruppe oder -Y-$CO_2^-$ für eine Sulfosuccinatgruppe steht; $R^1$ für eine gesättigte oder ungesättigte, verzweigte oder unverzweigte Kohlenwasserstoffgruppe, die mit Hydroxygruppen substituiert sein kann oder $R^1$-X für eine alkoxylierte oder nicht alkoxylierte Alkylcitratgruppe steht; $R^2$ und $R^3$ unabhängig voneinander für Wasserstoff oder für eine Methylgruppe stehen; n den Alkoxylierungsgrad bedeutet, der zwischen 0 und 20 liegt; M für ein oder mehrere, die negativen Ladungen des Anions neutralisierende Gegenionen steht und m die Wertigkeit des Gegenions M bedeutet.

4.  Mittel nach Anspruch 1, **dadurch gekennzeichnet, daß** Komponente (a1) ausgewählt ist aus Sulfosuccinaten, Sulfosuccinamaten, carboxylierten Fettalkoholethoxylaten oder carboxylierten Fettsäureamidethoxylaten oder deren Gemischen.

5.  Mittel nach Anspruch 1, **dadurch gekennzeichnet, daß** Komponente (a1) ausgewählt ist aus

    -   Verbindungen der allgemeinen Formel (II)

$$R^1\text{-X-}(CHR^2CHR^3O)_n\text{-C(O)-}CHR^4\text{-}CHR^5\text{-}CO_2^-\ 1/m\ M^{m+} \tag{II},$$

- Alkylcitraten der allgemeinen Formel (III)

$$R^6O\text{-}(CH2CH2O)_{n1}\text{-}CO\text{-}CH2\text{-}C(OH)(CO\text{-}(OCH2CH2)_{n2}OH)\text{-}CH2\text{-}CO\text{-}$$

$$(OCH2CH2)_{n3}\text{-}O\text{-}CO\text{-}CHR^4\text{-}CHR^5\text{-}CO_2^-\ 1/m\ M^{m+} \tag{III}$$

- Verbindungen der allgemeinen Formel (IV)

$$R^1\text{-X-}(CH2CH2O)_n\text{-}CH2\text{-}CO_2^-\ 1/m\ M^{m+} \tag{IV}$$

wobei $R^1$ und $R^6$ für gesättigte oder ungesättigte, verzweigte oder unverzweigte Kohlenwasserstoffgruppen mit 5 bis 20 Kohlenstoffatomen, die mit Hydroxygruppen substituiert sein können, stehen; $R^2$ und $R^3$ unabhängig voneinander für Wasserstoff oder für eine Methylgruppe stehen, wobei mindestens einer der Reste $R^2$ und $R^3$ Wasserstoff bedeutet; n den Alkoxylierungsgrad bedeutet, der zwischen 0 und 20 liegt; M für ein oder mehrere, die negativen Ladungen des Anions neutralisierende Gegenionen steht und m die Wertigkeit des Gegenions M bedeutet; -X- bei Formel (II) für -COO-, -CONH- oder - NH- und bei Formel (IV) für -COO-, -CONH- oder -Osteht; die Reste $R^4$ und $R^5$ unabhängig voneinander Wasserstoff oder $SO_3^-$ bedeuten, wobei mindestens einer der beiden Reste Wasserstoff bedeutet; n1, n2 und n3 den Alkoxylierungsgrad bedeuten, der jeweils zwischen 0 und 10 liegt.

6. Mittel nach einem der vorstehenden Ansprüche, **dadurch gekennzeichnet, daß** Komponente (a1) in einer Menge von 0,1 bis 30 Gewichtsprozent vorliegt.

7. Mittel nach einem der vorstehenden Ansprüche, **dadurch gekennzeichnet, daß** Komponente (a2) ausgewählt ist aus ethoxylierten Fettsäuren, ethoxylierten ein- oder mehrwertigen Alkoholen, ethoxyliertem, hydrierten oder nicht hydriertem Rizinusöl, Glyceridalkoxylaten, Fettsäureglyceridpolyalkylenglykolethern, Fettsäurepartialglyceridpolyalkylenglykolethern, Polyglykolamiden, ethoxylierten oder nicht ethoxylierten Fettsäurezuckerestern und Partialglyceriden.

8. Mittel nach einem der vorstehenden Ansprüche, **dadurch gekennzeichnet, daß** Komponente (a2) in einer Menge von 0,1 bis 40 Gewichtsprozent vorliegt.

9. Mittel nach einem der vorstehenden Ansprüche, **dadurch gekennzeichnet, daß** die Zusammensetzung (A) in einer transparenten und einphasigen Form vorliegt.

10. Mittel nach einem der vorstehenden Ansprüche, **dadurch gekennzeichnet, daß** es in einer transparenten Verpackung vorliegt.

11. Mittel nach einem der vorstehenden Ansprüche, **dadurch gekennzeichnet, daß** es zusätzlich mindestens einen mehrwertigen Alkohol enthält.

12. Mittel nach einem der vorstehenden Ansprüche, **dadurch gekennzeichnet, daß** es frei ist von chemischen Treibmitteln.

13. Verwendung eines anionischen, eine Carboxylatgruppe enthaltenden Tensids zur Herstellung einer transparenten, einphasigen, mittels einer mechanischen Vorrichtung verschäumbaren Zusammensetzung.

14. Verwendung nach Anspruch 13, **dadurch gekennzeichnet, daß** das eine Carboxylatgruppe enthaltende Tensid ein Sulfosuccinat-Tensid ist.

15. Verwendung nach Anspruch 13, **dadurch gekennzeichnet, daß** das eine Carboxylatgruppe enthaltende Tensid die allgemeine Formel (I) gemäß Anspruch 3 besitzt.

**Claims**

1. Hair-treatment agent consisting of

    (A) a single-phase composition comprising

        (a1) at least one anionic surfactant containing a carboxylate group and
        (a2) at least one nonionic emulsifier

    in combination with
    (B) a pump foam device for foaming the composition (A).

2. Agent according to Claim 1, **characterized in that** component (a1) is a sulfosucciniate surfactant.

3. Agent according to Claim 1, **characterized in that** component (a1) has the general formula (I)

$$R^1\text{-X-(CHR}^2\text{-CHR}^3\text{-O)}_n\text{-Y-CO}_2^-\ 1/m\ M^{m+} \tag{I}$$

where -X- is -COO-, -CONH-, -O- or -NH-; -Y- is an alkylene group or $-Y\text{-CO}_2^-$ is a sulfosuccinate group; $R^1$ is a saturated or unsaturated, branched or unbranched hydrocarbon group which may be substituted by hydroxyl groups, or $R^1$-X is an alkoxylated or non-alkoxylated alkyl citrate group; $R^2$ and $R^3$, independently of one another, are hydrogen or a methyl group; n is the degree of alkoxylation, which is between 0 and 20; M is one or more counterions neutralizing the negative charges of the anion, and m is the valency of the counterion M.

4. Agent according to Claim 1, **characterized in that** component (a1) is chosen from sulfosuccinates, sulfosuccinamates, carboxylated fatty alcohol ethoxylates or carboxylated fatty acid amide ethoxylates or mixtures thereof.

5. Agent according to Claim 1, **characterized in that** component (a1) is chosen from

    - compounds of the general formula (II)

$$R^1\text{-X-(CHR}^2\text{CHR}^3\text{O)}_n\text{-C(O)-CHR}^4\text{-CHR}^5\text{-CO}_2^-\ 1/m\ M^{m+} \tag{II}$$,

    - alkyl citrates of the general formula (III)

$$R^6\text{O(CH2CH2O)}_{n1}\text{-CO-CH2-C(OH)(CO- (OCH2CH2)}_{n2}\text{OH)-}$$

$$\text{CH2-CO-(OCH2CH2)}_{n3}\text{+-O-CO-CHR}^4\text{-CHR}^5\text{-CO}_2^-\ 1/m\ M^{m+} \tag{III}$$

    - compounds of the general formula (IV)

$$R^1\text{-X-(CH2CH2O)}_n\text{-CH2-CO}_2^-\ 1/m\ M^{m+} \tag{IV}$$

where $R^1$ and $R^6$ are saturated or unsaturated, branched or unbranched hydrocarbon groups having 5 to 20 carbon atoms, which may be substituted by hydroxyl groups; $R^2$ and $R^3$, independently of one another, are hydrogen or a methyl group; where at least one of the radicals $R^2$ and $R^3$ is hydrogen; n is the degree of alkoxylation, which is between 0 and 20; M is one or more counterions neutralizing the negative charges of the anion, and m is the valency of the counterion M; -X- in formula (II) is -COO-, -CONH- or - NH- and in formula (IV) is -COO-, -CONH- or -O-; the radicals $R^4$ and $R^5$, independently of one another, are hydrogen or $SO_3^-$, where at least one of the two radicals is hydrogen; n1, n2 and n3 are the degree of alkoxylation which in each case is between 0 and 10.

6. Agent according to one of the preceding claims, **characterized in that** component (a1) is present in an amount of from 0.1 to 30 percent by weight.

7. Agent according to one of the preceding claims, **characterized in that** component (a2) is chosen from ethoxylated fatty acids, ethoxylated mono- or polyhydric alcohols, ethoxylated, hydrogenated or nonhydrogenated castor oils, glyceride alkoxylates, fatty acid glyceride polyalkylene glycol ethers, fatty acid partial glyceride polyalkylene glycol ethers, polyglycol amides, ethoxylated and non-ethoxylated fatty acid sugar esters and partial glycerides.

8. Agent according to one of the preceding claims, **characterized in that** component (a2) is present in an amount of from 0.1 to 40 percent by weight.

9. Agent according to one of the preceding claims, **characterized in that** the composition (A) is present in a transparent and single-phase form.

10. Agent according to one of the preceding claims, **characterized in that** it is present in a transparent packaging.

11. Agent according to one of the preceding claims, **characterized in that** it additionally comprises at least one polyhydric alcohol.

12. Agent according to one of the preceding claims, **characterized in that** it is free from chemical propellants.

13. Use of an anionic surfactant containing a carboxylate group for the preparation of a transparent, single-phase composition foamable by means of a mechanical device.

14. Use according to Claim 13, **characterized in that** the surfactant containing a carboxylate group is a sulfosuccinate surfactant.

15. Use according to Claim 13, **characterized in that** the surfactant containing a carboxylate group has the general formula (I) according to Claim 3.

**Revendications**

1. Composition pour le traitement des cheveux, constituée de

    (A) une composition monophasique contenant

        (a1) au moins un tensioactif anionique contenant un groupe carboxylate et
        (a2) au moins un émulsifiant non ionique

    en association avec
    (B) un dispositif de moussage à pompe pour faire mousser la composition (A).

2. Composition selon la revendication 1, **caractérisée en ce que** le composant (a1) est un tensioactif sulfosuccinate.

3. Composition selon la revendication 1, **caractérisée en ce que** le composant (a1) présente la formule générale (I)

$$R^1\text{-X-}(CHR^2\text{-}CHR^3\text{-O})_n\text{-Y-}CO_2^- \ 1/m \ M^{m+} \tag{I}$$

dans laquelle -X- représente -COO-, -CONH-, -O- ou -NH- ;
-Y- représente un groupe alkylène ou -Y-$CO_2^-$ représente un groupe sulfosuccinate ; $R^1$ représente un groupe hydrocarboné saturé ou insaturé, ramifié ou non ramifié, qui peut être substitué par des groupes hydroxy, ou $R^1$-X représente un groupe alkylcitrate alcoxylé ou non alcoxylé ; $R^2$ et $R^3$ représentent, indépendamment l'un de l'autre, un atome d'hydrogène ou un groupe méthyle ; n représente le degré d'alcoxylation, qui est compris entre 0 et 20 ; M représente un ou plusieurs ions opposés neutralisant les charges négatives de l'anion et m représente la valence de l'ion opposé M.

4. Composition selon la revendication 1, **caractérisée en ce que** le composant (a1) est choisi parmi des sulfosuccinates, des sulfosuccinamates, des produits d'éthoxylation d'alcools gras carboxylés et des produits d'éthoxylation d'amides gras carboxylés ou des mélanges de ceux-ci.

5. Composition selon la revendication 1, **caractérisée en ce que** le composant (a1) est choisi parmi

   - des composés de formule générale (II)

$$R^1\text{-X- (CHR}^2\text{CHR}^3\text{O)}_n\text{-C(O)-CHR}^4\text{-CHR}^5\text{-CO}_2^-\ 1/m\ M^{m+} \qquad \text{(II),}$$

   - des alkylcitrates de formule générale (III)

$$R^6\text{O-(CH}_2\text{CH}_2\text{O)}_{n1}\text{-CO-CH}_2\text{-C(OH) (CO- (OCH}_2\text{CH}_2\text{)}_{n2}\text{OH)-CH}_2\text{-CO-}$$

$$\text{(OCH}_2\text{CH}_2\text{)}_{n3}\text{-O-CO-CHR}^4\text{-CHR}^5\text{-CO}_2^-\ 1/m\ M^{m+} \qquad \text{(III)}$$

   - des composés de formule générale (IV)

$$R^1\text{-X-(CH}_2\text{CH}_2\text{O)}_n\text{-CH}_2\text{-CO}_2^-\ 1/m\ M^{m+} \qquad \text{(IV)}$$

   Où $R^1$ et $R^6$ représentent des groupes hydrocarbonés saturés ou insaturés, ramifiés ou non ramifiés, ayant de 5 à 20 atomes de carbone, qui peuvent être substitués par des groupes hydroxy ; $R^2$ et $R^3$ représentent, indépendamment l'un de l'autre, un atome d'hydrogène ou un groupe méthyle, au moins l'un des radicaux $R^2$ et $R^3$ représentant un atome d'hydrogène ; n représente le degré d'alcoxylation, qui est compris entre 0 et 20 ; M représente un ou plusieurs ions opposés neutralisant les charges négatives de l'anion et m représente la valence de l'ion opposé M ; -X- représente dans la formule (II) -COO-, -CONH- ou - NH- et dans la formule (IV) -COO-, -CONH- ou -O-; les radicaux $R^4$ et $R^5$ représentent, indépendamment l'un de l'autre, un atome d'hydrogène ou $SO_3^-$, au moins l'un des deux radicaux représentant un atome d'hydrogène ; n1, n2 et n3 représentent le degré d'alcoxylation, qui est chaque fois compris entre 0 et 10.

6. Composition selon l'une quelconque des revendications précédentes, **caractérisée en ce que** le composant (a1) est présent en une quantité de 0,1 à 30 % en poids.

7. Composition selon l'une quelconque des revendications précédentes, **caractérisée en ce que** le composant (a2) est choisi parmi des acides gras éthoxylés, des alcools mono- ou polyhydroxylés éthoxylés, l'huile de ricin hydrogénée ou non hydrogénée, éthoxylée, des produits d'alcoxylation de glycérides, des glycéride-polyalkylèneglycoléthers d'acides gras, des glycéride-polyalkylèneglycoléthers partiels d'acides gras, des polyglycolamides, des esters glucidiques d'acides gras éthoxylés ou non éthoxylés et des glycérides partiels.

8. Composition selon l'une quelconque des revendications précédentes, **caractérisée en ce que** le composant (a2) est présent en une quantité de 0,1 à 40 % en poids.

9. Composition selon l'une quelconque des revendications précédentes, **caractérisée en ce que** la composition (A) se trouve sous une forme transparente et monophasique.

10. Composition selon l'une quelconque des revendications précédentes, **caractérisée en ce qu'**elle se trouve dans un conditionnement transparent.

11. Composition selon l'une quelconque des revendications précédentes, **caractérisée en ce qu'**elle contient en outre au moins un alcool polyhydroxylé.

12. Composition selon l'une quelconque des revendications précédentes, **caractérisée en ce qu'**elle est exempte de propulseurs chimiques.

**13.** Utilisation d'un tensioactif anionique, contenant un groupe carboxylate, pour la préparation d'une composition transparente monophasique pouvant être transformée en mousse au moyen d'un dispositif mécanique.

**14.** Utilisation selon la revendication 13, **caractérisée en ce que** le tensioactif contenant un groupe carboxylate est un tensioactif sulfosuccinate.

**15.** Utilisation selon la revendication 13, **caractérisée en ce que** le tensioactif contenant un groupe carboxylate possède la formule générale (I) selon la revendication 3.